# EUROPEAN PATENT SPECIFICATION

(11) **EP 2 691 058 B1**
(45) Date of publication and mention of the grant of the patent: **02.03.2016**
(21) Application number: 12717223.7
(22) Date of filing: 29.03.2012
(51) Int. Cl.: A61F 13/02

(54) **CUSHION FOR PATIENT INTERFACE**
KISSEN FÜR EINE PATIENTENSCHNITTSTELLE
COUSSIN POUR INTERFACE PATIENT

(30) Priority: 29.03.2011 EP 11160280
(43) Date of publication of application: 05.02.2014
(73) Proprietor: ResMed R&D Germany GmbH, 82152 Martinsried (DE)
(72) Inventor: LANG, Bernd, 82166 Gräfelfing (DE); NICKOL, Johannes, 80636 Munich (DE); ROTHFUSS, Jens, 80807 Munich (DE); BURZ, Sebastian, 87656 Germaringen (DE); KIRCHBERGER, Andreas, 83714 Miesbach (DE)
(74) Representative: Vossius & Partner Patentanwälte Rechtsanwälte mbB
(86) International application number: PCT/EP2012/055727
(87) International publication number: WO 2012/131001

(56) References cited:
- WO-A1-2009/062265
- US-A- 5 770 220

## Description

The present invention relates to a cushion for a patient interface, the patient interface being preferably used in a treatment of, e.g., sleep disorder breathing (SDB) with non-invasive positive pressure ventilation (NPPV).

The use of NPPV for treatment of SDB such as obstructive sleep apnea (OSA) was pioneered by Sullivan (see US 4,944,310). Apparatus for treatment of SDB generally involves a blower, which delivers a supply of positive pressure to a patient interface via a conduit. A patient interface may take several forms, such as a nasal mask assembly and a nasal and mouth mask assembly. Patients typically wear a mask assembly while sleeping to receive the NPPV therapy.

Patient interfaces or mask assemblies typically comprise a rigid shell or frame and a soft face-contacting cushion. The cushion spaces the frame away from the patient's face. The frame and cushion define a cavity which receives the nose and/or mouth of the patient. The frame and cushion are generally held in position on the patient's face by a headgear assembly. The headgear assembly typically comprises an arrangement of straps which pass along both sides of the patient's face to the back or crown of the patient's head.

Since this kind of treatment often has to continue throughout the entire rest or sleep phase, the interface has to be as comforting to the patient or user as possible and to be adapted for use over an extended period of time. Patient interfaces, such as respiratory masks, therefore often comprise a cushion, e.g., an elastomer, foam or gel cushion, whose purpose it is to provide a soft and comforting contact area or interface between the skin of the patient's face and the patient interface. Such cushions may also establish a seal between the mask and the patient's skin so that the mask interior is sealed from ambiance. This is of relevance particularly in the field of, e.g., CPAP or BPAP, breathing therapy where breathable gas is supplied at elevated pressures. The provision of a tight seal and avoidance of leaks is important to achieve a reliable and predictable breathing pressure and thus effective therapy. These cushions need to be soft and cushy on the one hand yet have to provide enough stability and rigidity on the other hand to keep the mask in place and to establish the required seal.

Many attempts have been made in the art to adapt interfaces such as face masks and cushions for face masks in a way to improve patient comfort and therapy compliance. For example, patient interfaces have been adapted to be properly aligned with the patient's physiognomy and structure of patient's faces, particularly for long term compliance with the applied therapy. In this regard, it has been observed that, particularly due to the pressure with which a patient interface has to be held on a patient's face to, e.g., sealingly contact a patient's face and to allow the provision of breathing therapy, the patient's skin being in contact with the patient interface may be affected.

Mostly, attempts have been made to, e.g., provide a beneficial and comforting structure of the interface cushion or to adapt the breathing pressures involved to the minimum required for an effective therapy.

US 7,406,966 discloses a nasal interface kit comprising amongst separately arranged and packed components a mask, a cleaning and storage container, and a skin ointment.

However, devices known in the prior art are still disadvantageous in that their contact surfaces which are pressed onto user's skin may lead to discomfort or even reddening or pressure marks on the user's skin, sometimes including pain. The user's skin can also be adversely affected leading to wounds and/or inflammation.

It is thus an aspect of the present technology to overcome the problems of the prior art and, in particular, to provide a patient interface which provides excellent functionality, particularly with regards to comfort and use in long-term therapy. Particularly, an aspect of the present technology is to provide a patient interface which avoids or at least ameliorates the disadvantages of the prior art. Preferably, the device according to the present technology contacts the user's skin, without causing any harm to the user's skin, even during long-term therapy and may make the patient feel comfortable.

The present technology is achieved by the features of independent claims wherein the dependent claims are directed to preferred embodiments of the present technology.

According to the present technology, there is provided a patient interface, particularly a patient interface cushion, which during use releases one or more substances to a patient. The release of the substance(s) is preferably controlled and/or sustained. Particularly, depending on the nature of the substance and the intended use, in order to achieve best compliance. The patient interface may be a breathing mask for applying breathing therapy, e.g. CPAP and/or BPAP therapy.

Preferably, these substances improve the patient's compliance with wearing a patient interface and/or to alleviate the influences of a patient interface being worn over a long period, such as a couple of hours, days and/or months. Preferably, during contact with the patient's skin, such substances, preferably skin care substances, are set free or migrate from the mask and reach the patient's skin.

The patient interface preferably comprises a member for contacting the skin of a patient during use. Such member or at least a part of said member comprises the substance. The member and/or substance is/are adapted to release, preferably by sustained and/controlled release, the substance, thereby preferably allowing the substance to contact the patient's skin.

The member for contacting the patient's skin is preferably a mask cushion, preferably a nasal and/or mouth cushion and/or a mask pad. The member may also be a forehead pad and/or a headband means, or at least a part thereof or associated therewith, e.g. a yoke, rigidiser or stiffening portion or any portion contacting a patient's skin.

Preferably, the cushion is made of a first material, wherein the substance is or comprises a second material. According to a preferred embodiment, the member, preferably cushion, is made of a silicone material. The silicone material may be a silicone foam and/or a silicone structure, such as a bladder or a structure comprising one or more pockets, preferably containing a second substance. This second substance preferably is or comprises silicone gel and/or water or a water based structure. In particular, it has been found out that a silicone bladder or pocket allows a sustained release of water or a water based substance contained therein. This also applies to oils or oil like substances contained in the silicone material or in a pocket or cavity closed by a silicone material. According to further preferred embodiments, the first material and/or the member comprise thermoplastic elastomer (TPE), polyurethane (PU), nylon and/or the like. As discussed above with regard to silicone, such material may equally be a foam structure etc.

The second material or substance is or comprises a skin care component, ointment and/or a medical or therapeutical component, preferably for enhancing wound healing and/or alleviating pressure marks, reddenings and/or inflammation of/on a patient's skin. Such skin care components per se are known in the art. Such known skin care substances may be made on an oil base, as known in the art. According to an aspect of the present technology, silicone oil known in production of silicone products may be used as such oil base for a skin care substance.

The physical or chemical process to release the substance can be manifold and preferably includes one or more of the mechanisms described herein. For example the second substance may comprise or be a silicone material such as a so called silicone oil or an oily, silicone based substance which is sustainably released when contained in a silicone material or in a pocket or cavity closed by a silicone material. The first member may also comprise a bladder or one or more pockets enclosed by silicone material wherein the second substance contained in the cavity or pocket is or comprises water or a water based substance.

According to a preferred additional or alternative solution, the member is or comprises a material, preferably silicone, and more preferably silicone, foam which includes a second substance, preferably a so called silicone oil or an oily, silicone based substance, preferably a free silicone compound which is not chemically bound in the first material.

Here, the first material is preferably silicone. Silicone may be molded to the shape of the patient interface member. Preferred silicone may be a two component silicone which crosslinks as a reaction of the two components (e.g. first component A and a second component B) being brought together, particularly with a specific ratio, eventually along with environmental conditions such as temperature. Here, the second substance, preferably a so called silicone oil, may have been added to the first component A and/or the second component B prior to these components reacting to become the first material. Alternatively or in addition, the second material or substance may be added to first component A and second component B as a third component during production. Preferably, the second substance does not contribute to the (chemical) reaction of the first and second component with one another.

According to this, the second material is contained in the structure of the first material, preferably while being chemically unbound or free to migrate out of the material, as further explained herein. It is preferably part of the material per se. It is preferably not contained in pores, cavities or pockets of the material.

As indicated above, the second material may be foam, preferably a silicone foam. As is well known, foam is produced by use of some foaming or expanding agent which may be added to first component A and/or second component B prior to or during molding. As discussed above, the second substance may have been added to the first component A and/or the second component B prior to these components reacting to become the first material. Alternatively or in addition, the second material or substance may be added to first component A and second component B as a third component during production. Preferably, the foam is closed cell foam. Preferably, the second substance does neither contribute to the (chemical) reaction of the first and second component with one another nor to the foaming process.

Again, the second material is contained in the structure of the first material, preferably while being chemically unbound or free to migrate our of the material, as further explained herein. It is preferably part of the material per se. It is preferably not contained in pores, cavities or pockets of the foamed material.

The second material may be substantially evenly distributed throughout the first material. Preferably, all surfaces of the first material release the second substance. Should it be desired to prevent the second material to be released at specific locations, according to a preferred aspect a mechanical barrier can be foreseen. Such mechanical barrier may take the form of a thin film not comprising the second substance or being substantially impermeable to the second substance. According to another alternative or additional aspect of the present technology, the first material may be a patch or a foil which can be applied, e.g. adhered or bonded, to a patient interface. Thereby, the advantages of the present technology can be applied to existing products or products not exhibiting the advantageous features of the present technology.

The manufacturing process may be compared to paint, e.g. pigments, being added to a material prior to or during production.

Preferably, the amount of the second substance is up to about 10% of the weight of the first material or member, and preferably is about 1 to 9%, and also preferably about 2 to 8%. The higher the amount of the second substance, the higher the amount of substance released to the patient's skin. The effect discussed herein can be easily shown by use of blotting paper on which the first material (member) is laid.

The first material releases the second substance, i.a., the first material exudes the substance, the substance migrates from the first material, the first material presses out the substance, the substance capillary moves out of the first material, and/or the first material functions as a membrane allowing the substance to permeate therethrough during use. In case of the first material being a foam, preferably a silicone foam, the second substance may be contained in the pores of the foam. Alternatively and also preferred, particularly, i.a., in view of ease of manufacturing, the second substance is not contained in the pores but in the material itself. Effects for releasing the second substance may thus include migration, concentration differences, capillary forces, membrane effects, pressure etc.

As also indicated above, at least part of the contact surfaces of a first member and particularly of a cushion of the patient interface is made of a silicone material. Such material may be a silicone rubber, a silicone foam, and/or a silicone gel. This silicone material, preferably silicone foam, contains additional substances which can be released or which are released to the environment, and preferably patient's skin during use of the mask. The substances may be, according to preferred embodiments, oils, water based substances, silicone oils, and/or substances or oils used in skin-care creams. According to a preferred aspect, the skin contacting material of the patient interface releases or sweats out the additional substance contained therein under preferably ordinary conditions such as ambient pressure and room temperature. According to a preferred further aspect, the substances are sweated out or are exuded by the interface when the contact region or cushion is put under pressure while being worn by a patient.

According to a preferred aspect, the second substance is based on silicone oil as referred to above and as known to be used for manufacturing silicone products. Such silicone oil may form the basis of or act as a carrier for a care substance.

Further examples are contact surfaces of patient interfaces made of silicone parts, e.g., having a hardness in the range from gel-like hardness to Shore80A, depending on the desired use, which sweat out skin-care substances, preferably based on silicone oil, and thus release them to the patient's skin during use. While a lower hardness may be desired for face cushions, a higher hardness may be desired for parts, e.g. rigid or supporting parts, of a headgear.

According to a preferred embodiment, the discussed release of the second substance from the skin contacting material exhausts or runs out over time, preferably allowing the definition and/or adjusting of a defined life-time of the advantageous effect of the exuding skin-contacting material. Preferably, the patient interface cushion can still be used even after it does no longer exude the advantageous and preferred second substance. Preferably, the second substance is released from the first material for a period not more than about one month, preferably for a period of not more than about two months, four months, six months or twelve months. The present technology may allow this period to be visualized by the second substance being adapted to influence the colour or colour grade of the first material. Preferably, the second substance is coloured such that its presence in the first material is visible, e.g. the first material my look darker or have a different colour with the second substance being present therein than without the second substance.

Preferably, the second material is already mixed with or applied to the contact material of the patient interface during manufacturing. According to a preferred embodiment, the silicone oil as used for producing a silicone cushion comprises added care substances or the like in order to achieve beneficial effects on a patient's skin. Preferably, the first material is not impregnated with the second substance. Rather, the second substance is admixed with the first material and/or forms a compound thereof.

The present invention allows avoidance and reduction of negative effects of long-time therapy and long-time wearing of a patient interface such as face, nose and/or mouth masks. If not overcome, pressure marks caused by face masks can escalate to open wounds if the masks are used on a day-to-day basis. However, also in this case, substances that enhance wound healing are conceivable and may advantageously be used with the present invention.

While the technology has been illustrated and described in detail in the drawings and foregoing description, such illustration and description are to be considered illustrative or exemplary and non-restrictive; the invention is thus not limited to the disclosed aspects. Variations to the disclosed aspects can be understood and effected by those skilled in the art and practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The disclosed technology also relates to the exact terms, features, numerical values or ranges if the specification above refers to these together with expressions such as 'about', 'substantially', 'at least' and vice versa. For example, "about three" is understood to encompass "3" and "exactly 3" while "substantially radial" is understood to encompass "radial" and "exactly radial". The mere fact that certain measures are recited in mutually different dependent claims does not indicate that a combination of these measures can not be used to advantage. Any reference signs in the claims should not be considered as limiting the scope.

## Claims

1. Mask cushion for a breathing mask, the breathing mask comprising a frame and particularly being adapted to be used in treatment of sleep disordered breathing, the mask cushion being for contacting the skin of a patient during use, **characterized in that** at least a part of said mask cushion comprises a substance, wherein the mask cushion is adapted to release the substances to the patient's skin during use, and wherein
the substance is or comprises oils, water based substances, silicone oils possibly acting as a carrier for a care substance, and/or substances or oils used in skin-care creams, and wherein
the mask cushion is made of a first material and the first material is adapted to exude the substance, wherein the substance migrates from the first material, wherein the first material is adapted to press out the substance by being put under pressure while being worn by a patient, wherein the substance moves out of the first material through a capillary,
wherein the substance moves out of the first material due to concentration differences and/or wherein the first material functions as a membrane allowing the substance to permeate therethrough during use, wherein the substance is or comprises a second material, the second material being a care substance comprising silicone oil.

2. Mask cushion according to claim 1, wherein the substance is or comprises a skin care component, ointment and/or a medical or therapeutical component, preferably for enhancing wound healing and/or alleviating pressure marks, reddening and/or inflammation of/on a patient's skin and/or improving the patient's compliance with wearing a patient interface.

3. Mask cushion according to claim 1, wherein the mask cushion for contacting the patient's skin is a nasal and/or mouth cushions.

4. Mask cushion according to any one of the preceding claims, wherein the release of the substance is a sustained and/or controlled release.

5. Mask cushion according to any one of the preceding claims, wherein the substance is or comprises a second material.

6. Mask cushion according to any one of the preceding claims, wherein the mask cushion is made of a silicone, a thermoplastic elastomer (TPE), a polyurethane (PU) and/or a nylon material.

7. Mask cushion according to any one of the preceding claims, wherein the first material is a foam, preferably a silicone foam and/or a structure, preferably a silicone structure, such as a pocket or bladder, containing a gel, preferably a silicone gel, and/or water or a water based structure.

8. Breathing mask comprising a mask cushion according to any one of the preceding claims.

9. Breathing mask according to claim 8, wherein the breathing mask is a breathing mask for applying breathing therapy, preferably CPAP and/or BPAP therapy.

10. A method of manufacturing a mask cushion for a breathing mask and preferably for a patient contacting portion, the method comprising the steps
providing a first material and a second material, the first material being silicone, preferably a two-component silicone, the second material being a care substance comprising silicone oil,
molding the mask cushion from the first and second material.

11. Method according to claim 10, wherein the mask cushion is according to any one of claims 1 to 9.

12. Method according to claim 10 or 11, wherein the breathing mask is according to any one of claims 8 or 9.

13. Method according to claim 10, 11 or 12 wherein the method comprises the steps of providing the first material with the second substance admixed thereto, preferably the second material being admixed to the first component and/or second component of the first material; or the step of
admixing the second substance with the first material, preferably with the first component and/or the second component prior to and/or after mixing the first and second components.

14. Method according to any one of claims 10 to 13, further comprising the step of adding a foaming agent.

## Patentansprüche

1. Maskenkissen für eine Atemmaske, wobei die Atemmaske einen Rahmen aufweist und besonders geeignet ist, bei der Behandlung schlafbezogener Atmungsstörungen verwendet zu werden, und das Maskenkissen zum Kontaktieren der Haut eines Patienten im Gebrauch dient, **dadurch gekennzeichnet, dass** mindestens ein Teil des Maskenkissens eine Substanz aufweist, wobei das Maskenkissen geeignet ist, die Substanz zur Haut des Patienten im Gebrauch freizusetzen, und wobei die Substanz Öle, Substanzen auf Wasserbasis, möglicherweise als Träger für eine Pflegesubstanz wirkende Silikonöle und/oder in Hauptpflegecremes verwendete Substanzen oder Öle ist oder aufweist und wobei
das Maskenkissen aus einem ersten Material hergestellt ist und das erste Material geeignet ist, die Substanz abzusondern, wobei die Substanz aus dem ersten Material wandert, wobei das erste Material geeignet ist, die Substanz auszupressen, indem es unter Druck gesetzt wird, während es von einem Patienten getragen wird, wobei sich die Substanz aus dem ersten Material durch eine Kapillare bewegt, wobei sich die Substanz aus dem ersten Material infolge von Konzentrationsdifferenzen bewegt und/oder wobei das erste Material als Membran fungiert, die ermöglicht, dass die Substanz sie im Gebrauch durchdringt, wobei die Substanz ein zweites Material ist oder aufweist, wobei das zweite Material eine Silikonöl aufweisende Pflegesubstanz ist.

2. Maskenkissen nach Anspruch 1, wobei die Substanz eine Hautpflegekomponente, Salbe und/oder eine medizinische oder therapeutische Komponente ist oder aufweist, vorzugsweise zur Beschleunigung der Wundheilung und/oder Linderung von Druckstellen, Rötung und/oder Entzündung der/auf der Haut eines Patienten und/ oder Verbesserung der Compliance des Patienten beim Tragen einer Patientenschnittstelle.

3. Maskenkissen nach Anspruch 1, wobei das Maskenkissen zum Kontaktieren der Haut des Patienten ein Nasen- und/oder Mundkissen ist.

4. Maskenkissen nach einem der vorstehenden Ansprüche, wobei die Freisetzung der Substanz eine langzeitig wirkende und/oder gesteuerte Freisetzung ist.

5. Maskenkissen nach einem der vorstehenden Ansprüche, wobei die Substanz ein zweites Material ist oder aufweist.

6. Maskenkissen nach einem der vorstehenden Ansprüche, wobei das Maskenkissen aus einem Silikon-, einem thermoplastischen Elastomer- (TPE), einem Polyurethan-(PU) und/oder einem Nylonmaterial hergestellt ist.

7. Maskenkissen nach einem der vorstehenden Ansprüche, wobei das erste Material ein Schaumstoff, vorzugsweise ein Silikonschaumstoff, und/oder eine Struktur, vorzugsweise eine Silikonstruktur, z. B. eine Tasche oder Blase, die ein Gel enthält, vorzugsweise ein Silikongel, und/oder Wasser oder eine Struktur auf Wasserbasis ist.

8. Atemmaske, die ein Maskenkissen nach einem der vorstehenden Ansprüche aufweist.

9. Atemmaske nach Anspruch 8, wobei die Atemmaske eine Atemmaske zur Anwendung von Atemtherapie ist, vorzugsweise CPAP- und/oder BPAP-Therapie.

10. Verfahren zur Herstellung eines Maskenkissens für eine Atemmaske und vorzugsweise für einen den Patienten kontaktierenden Abschnitt, wobei das Verfahren die Schritte aufweist:
Bereitstellen eines ersten Materials und eines zweiten Materials, wobei das erste Material Silikon ist, vorzugsweise ein Zweikomponentensilikon, und das zweite Material eine Silikonöl aufweisende Pflegesubstanz ist,
Formen des Maskenkissens aus dem ersten und zweiten Material.

11. Verfahren nach Anspruch 10, wobei das Maskenkissen eines nach einem der Ansprüche 1 bis 9 ist.

12. Verfahren nach Anspruch 10 oder 11, wobei die Atemmaske eine nach Anspruch 8 oder 9 ist.

13. Verfahren nach Anspruch 10, 11 oder 12, wobei das Verfahren aufweist: die Schritte des Bereitstellens des ersten Materials mit der ihm beigemischten zweiten Substanz, wobei das zweite Material vorzugsweise der ersten Komponente und/oder zweiten Komponente des ersten Materials beigemischt ist; oder den Schritt des Beimischens der zweiten Substanz dem ersten Material, vorzugsweise der ersten Komponente und/oder der zweiten Komponente vor und/oder nach Mischen der ersten und zweiten Komponente.

14. Verfahren nach einem der Ansprüche 10 bis 13, das ferner den Schritt des Zugebens eines Schaummittels aufweist.

## Revendications

1. Coussinet de masque pour un masque respiratoire, le masque respiratoire comprenant un cadre et étant en particulier adapté pour être utilisé dans le traitement d'un trouble respiratoire du sommeil, le coussinet de masque étant destiné à être mis en contact avec la peau d'un patient en cours d'utilisation, **caractérisé en ce qu'**au moins une partie dudit coussinet de masque comprend une substance, dans lequel le coussinet de masque est conçu pour libérer la substance vers la peau du patient en cours d'utilisation, et dans lequel
la substance est ou contient de l'huile, des substances à base d'eau, des huiles de silicone agissant éventuellement comme un support pour une substance de soin, et/ou des substances ou huiles utilisées dans des crèmes de soin de la peau, et dans lequel le coussinet de masque est constitué d'un premier matériau et le premier matériau est conçu pour exsuder la substance, dans lequel la substance migre à partir du premier matériau, dans lequel le premier matériau est adapté pour expulser la substance en la plaçant sous pression alors qu'il est porté par un patient, dans lequel la substance sort du premier matériau à travers un capillaire, dans lequel la substance sort du premier matériau en raison de différences de concentration et/ou dans lequel le premier matériau sert de membrane, permettant à la substance de pénétrer à travers elle lors de l'utilisation, dans lequel la substance est ou comprend un second matériau, le second matériau étant une substance de soin comprenant de l'huile de silicone.

2. Coussinet de masque selon la revendication 1, dans lequel la substance est ou comprend un composant de soin de la peau, une pommade et/ou un composant médical ou thérapeutique, de préférence pour améliorer la cicatrisation des plaies et/ou soulager les marques de pression, rougeurs et/ou inflammations de/sur la peau d'un patient et/ou améliorer la tolérance par le patient du port d'une interface de patient.

3. Coussinet de masque selon la revendication 1, dans lequel le coussinet de masque destiné à entrer en contact avec la peau du patient est un coussinet nasal et/ou buccal.

4. Coussinet de masque selon l'une quelconque des revendications précédentes, dans lequel la libération de la substance est une libération prolongée et/ou contrôlée.

5. Coussinet de masque selon l'une quelconque des revendications précédentes, dans lequel la substance est ou comprend un second matériau.

6. Coussinet de masse selon l'une quelconque des revendications précédentes, dans lequel le coussinet de masque est constitué d'un silicone, d'un élastomère thermoplastique (TPE), d'un polyuréthane (PU) et/ou d'un matériau de nylon.

7. Coussinet de masse selon l'une quelconque des revendications précédentes, dans lequel le premier matériau est une mousse, de préférence, une mousse de silicone et/ou une structure, de préférence, une structure de silicone, telle qu'une poche ou une vessie, contenant un gel, de préférence, un gel de silicone, et/ou de l'eau ou une structure à base d'eau.

8. Masque respiratoire comprenant un coussinet de masque selon l'une quelconque des revendications précédentes.

9. Masque respiratoire selon la revendication 8, dans lequel le masque respiratoire est un masque respiratoire pour appliquer une thérapie respiratoire, de préférence, une thérapie CPAP et/ou BPAP.

10. Procédé de fabrication d'un coussinet de masque pour un masque respiratoire et de préférence, pour une partie en contact avec le patient, le procédé comprenant les étapes suivantes :
la fourniture d'un premier matériau et d'un second matériau, le premier matériau étant du silicone, de préférence du silicone à deux composants, le second matériau étant une substance de soin comprenant de l'huile de silicone,
le moulage du coussinet de masque à partir des premier et second matériaux.

11. Procédé selon la revendication 10, dans lequel le coussinet de masque est selon l'une quelconque des revendications 1 à 9.

12. Procédé selon la revendication 10 ou 11, dans lequel le masque respiratoire est selon l'une quelconque des revendications 8 et 9.

13. Procédé selon la revendication 10, 11 ou 12, dans lequel le procédé comprend les étapes de
fourniture du premier matériau auquel est mélangée la seconde substance, le second matériau étant mélangé de préférence au premier composant et/ou au second composant du premier matériau ; ou l'étape de
mélange de la seconde substance avec le premier matériau, de préférence avec le premier composant et/ou le second composant avant et/ou après mélange des premier et second composants.

14. Procédé selon l'une quelconque des revendications 10 à 13, comprenant en outre l'étape d'ajout d'un agent moussant.
